# EUROPEAN PATENT APPLICATION

(11) **EP 4 356 889 A1**
(43) Date of publication of application: **24.04.2024**
(21) Application number: 22201991.1
(22) Date of filing: 17.10.2022
(51) Int. Cl.: A61F 13/475, A61F 13/49, A61F 13/494

(54) **ABSORBENT ARTICLES HAVING A CONTAINMENT POCKET**

(71) Applicant: Ontex BV, 9255 Buggenhout (BE); Ontex Group NV, 9320 Erembodegem (BE)
(72) Inventor: INGENFELD, Björn, 53115 Bonn (DE); PANNWITT, Stefanie, 56727 Mayen (DE); LAMBERTZ, Christina, 56566 Neuwied (DE); BRAUN STREB, Hanne, 50389 Wesseling (DE); MOHAMMED, Moula Saheb, 56727 Mayen (DE)
(74) Representative: Larangé, Françoise

(57) **Abstract**

The present disclosure relates to absorbent articles having a containment pocket to hold body exudates. The absorbent articles chassis includes a topsheet, a backsheet joined to the topsheet, and an absorbent system located between the topsheet and the backsheet; it has a front waist region, a back waist region, and a crotch region located between the front waist region and the back waist region; it has a longitudinal axis extending through the front and back waist regions and a transverse axis substantially perpendicular to the longitudinal axis. The periphery of the chassis is defined by longitudinal outer edges and lateral outer edges; the periphery of the absorbent system is defined by longitudinal side edges and lateral side edges. The absorbent article further comprises barrier cuffs. Each barrier cuff distal edge is attached to the topsheet at a tack-down region in the front waist region and in the back waist region. In at least one of the front and back waist regions, a ratio R between the length A of the tack-down region measured parallel to the longitudinal axis in said waist region and the distance B between the lateral outer edge of the chassis and the lateral side edge of the absorbent system measured parallel to the longitudinal axis in said waist region is in the range 0.05 to 0.95.

## Description

### TECHNICAL FIELD

The present disclosure pertains to the technical field of absorbent hygiene products that can be used as articles for absorbing body fluids and exudates, such as urine and fecal material, or blood, menses, and vaginal fluids. In particular, the present disclosure relates to absorbent garments (or articles), such as disposable diapers or diaper pants, disposable incontinence diapers or pants, which are configured to collect and contain urine and fecal material and avoid leakage.

### BACKGROUND

One typical form of leakage is for exudates to leak out of the rear waist region or the front waist region of an absorbent article. As one example, fecal material that is not absorbed or contained by the absorbent article can move past the gaps between the absorbent article and the wearer's skin in the rear waist region and soil or contaminate the wearer's skin and clothing near his back. This may be more common of an occurrence for semi-solid fecal material, such as low viscosity fecal material, which can be prevalent with younger children. Such exudates can move around on the bodyside liner of an absorbent article under the influence of gravity, motion, and pressure by the wearer of the absorbent article. In such a circumstance, not only does the wearer's absorbent article need to be changed, but the wearer's clothing and/or bedding often also needs to be changed, resulting in additional work, expense, and stress for the caregiver.

One solution has been to provide absorbent articles with elastic waist features to ensure a more snugly fit of the article about the waist of the wearer and in particular avoiding any gap between the skin and the article in the back waist region. Elastic waist features will typically include an elasticized waistband consisting of an elastic member contractibly affixed between the topsheet and the backsheet. The elasticized waistband is designed to expand and contract with the wearer's motions and to maintain the fit of the absorbent article about the waist of the wearer during use. However this has revealed often not enough to retain exudates within the article. An example of an elastic waistband member is described in EP3400919.

Attempts have then been made to provide containment systems, especially on the bodyside liner or near the rear waist region, creating a void volume to hold body exudates until the absorbent core can absorb the exudates or until the article can be changed. One example is by providing a waist elastic member and not adhering a portion of it closest to the transverse axis of the absorbent article to the bodyside liner, such that the non-adhered portion of the waist elastic member can provide a containment pocket for exudates. EP3400919, for example, describes the combination of a transversal barrier cuff forming a pocket, and an elastic waistband member. Another example, described in WO 2016/159981 A1, provides a waist containment member disposed on the body facing surface of the chassis, including three folds to have an increased void volume to hold more body exudates or for longer. Although absorbent articles with such containment systems intend to prevent leakage of exudates, failures can still occur and above all, their construction and manufacture can become complex, requiring additional pieces of material and complicated manufacturing steps necessitating correct positioning and assembly in some regions whilst ensuring free parts in others, thereby also increasing manufacturing costs.

Thus, there remains a need to provide containment systems having simpler and/or less costly construction, whilst retaining or improving high performance against leakage of exudates, especially in the waist regions of the absorbent article.

### SUMMARY

The present disclosure relates to an absorbent article comprising a chassis including a topsheet, a backsheet joined to the topsheet, and an absorbent system located between the topsheet and the backsheet. The chassis has a front waist region, a back waist region, and a crotch region located between the front waist region and the back waist region. The chassis has a longitudinal axis extending through the front and back waist regions and a transverse axis substantially perpendicular to the longitudinal axis. The periphery of the chassis is defined by longitudinal outer edges and lateral outer edges; the periphery of the absorbent system is defined by longitudinal side edges and lateral side edges. The absorbent article comprises barrier cuffs extending in a longitudinal direction from the front waist region to the back waist region along the topsheet, each barrier cuff including a front end at the front waist region, a back end at the back waist region and proximal and distal edges connecting the front end and the back end. Each barrier cuff distal edge is attached to the topsheet at a tack-down region in the front waist region and in the back waist region. According to the present disclosure, in at least one of the front and back waist regions, a ratio R between the length A of the tack-down region measured parallel to the longitudinal axis in said waist region and the distance B between the lateral outer edge of the chassis and the lateral side edge of the absorbent system measured parallel to the longitudinal axis in said waist region is in the range 0.05 to 0.95, all measurements being made whilst the absorbent article is in a stretched, laid-flat configuration.

We have found that ensuring a sufficient zone without absorbent material between the absorbent system and the tack-down regions may offer an absorbent article having a performant and easy-to-manufacture containment pocket, without the need to incorporate any additional element.

Once worn, the region lying beyond the absorbent system until the tack-down regions and bordered by the raised barried cuffs and the waist region that is in contact with the user's skin is offering a containment pocket able to reliably retain exudates.

Other objects and advantages will become apparent hereinafter.

### BRIEF DESCRIPTION OF THE FIGURE

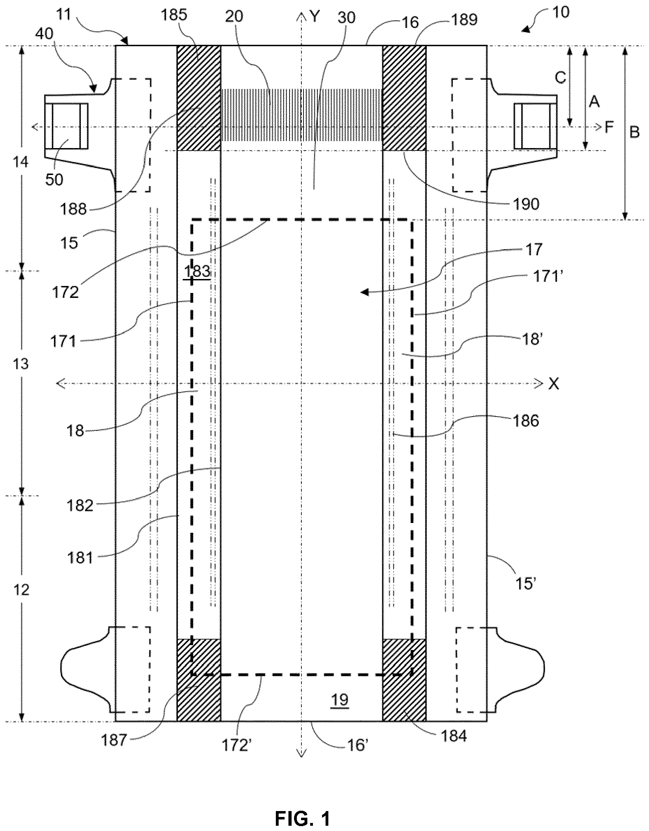

Fig. 1 is a schematic plan view of an absorbent article embodiment.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of' as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. An absorbent article, such as a diaper, comprises a liquid permeable "topsheet", a liquid impermeable "backsheet", and an "absorbent medium" disposed between the topsheet and the backsheet.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material. The absorbent core is generally wrapped into one or more core wrap layers, generally permeable nonwoven layers, enclosing the absorbent material to contain absorbent material therein and avoid leakage towards a skin of a wearer.

The term "backsheet" refers to a material forming the outer cover of the absorbent article. The backsheet prevents the exudates contained in the absorbent structure from wetting articles such as bedsheets and overgarments which contact the disposable absorbent article. The backsheet may be a unitary layer of material or may be a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials. The backsheet generally include, as outermost layer on the garment facing surface, a nonwoven material.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn. The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibres, cellulose acetate, cellulose triacetate, rayon, thermomechanical wood pulp, chemical wood pulp, debonded chemical wood pulp, milkweed, or bacterial cellulose.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition, adhesive to bond layers together.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

The term "disposed" is used to mean that an element(s) is formed (joined and positioned) in a particular place or position as a unitary structure with other elements or as a separate element joined to another element.

The term "joined" refers to configurations whereby an element is directly secured to another element by attaching the element directly to the other element, and configurations whereby an element is indirectly secured to another element by attaching the element to intermediate member(s) which in turn are attached to the other element. "Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension, i.e. the length, of the article and when combined with the term "substantially" includes directions within ±45° of the longitudinal direction.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels may be formed by joining upper and lower layers of a core wrap.

The term "topsheet" or "bodyside liner" refers to a liquid permeable material sheet forming the inner cover of the absorbent article and which in use is placed in direct contact with the skin of the wearer. The topsheet is typically employed to help isolate the wearer's skin from liquids held in the absorbent structure. The topsheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibres, such as wood pulp or cotton fibres, or from a mixture of natural and man-made fibres. Further examples of topsheet materials are porous foams, apertured plastic films, laminates of nonwoven materials and apertured plastic films etc. The materials suited as topsheet materials should be soft and non-irritating to the skin and be readily penetrated by body fluid

Embodiments of the articles according to the disclosure will now be described. It is understood that technical features described in one or more embodiments may be combined with one or more other embodiments without departing from the intention of the disclosure and without generalization therefrom, especially when such combinations are explicitly or implicitly inferred.

Referring to FIG. 1, a non-limiting embodiment of an absorbent article 10, for example a diaper, is illustrated. Other embodiments of the absorbent article could include pants and adult incontinence garments for example. FIG. 1 is a plan view of the absorbent article 10 in a flat-out, uncontracted state, otherwise stated, in a stretched, laid-flat configuration, i.e. without elastic induced contraction, with the portion of the absorbent article 10 which contacts the wearer facing the viewer.

The absorbent article 10 illustrated in FIG. 1 includes a chassis 11 which includes a front waist region 12, a back waist region 14, and a crotch region 13 disposed between the front waist region 12 and the back waist region 14 and interconnecting the front and back waist regions 12,14 respectively. The chassis 11 additionally has a longitudinal axis Y (also called "centreline" when dividing into two equal parts) extending through the front and back waist regions 12,14 and a transverse axis X (also called "centreline" when dividing into two equal parts) substantially perpendicular to the longitudinal axis Y. The chassis has a pair of longitudinal outer edges 15,15', and a pair of lateral outer edges 16,16', defining its periphery. The front waist region 12 may include the portion of the absorbent article 10 that, when worn, is positioned at least in part on the front of the wearer, while the back waist region 14 may include the portion of the absorbent article 10 that, when worn, is positioned at least in part on the back of the wearer. The crotch region 13 of the absorbent article 10 may include the portion of the absorbent article 10 that, when worn, is positioned between the legs of the wearer and can partially cover the lower torso of the wearer. The lateral outer edges 16,16' may be configured to encircle the waist of the wearer and together define a central waist opening for the waist of the wearer. Portions of the longitudinal outer edges 15,15' in the crotch region 13 may generally define leg openings for the legs of the wearer when the absorbent article 10 is worn.

The chassis 11 of the diaper 10 has an outer covering including a liquid permeable topsheet 19, a liquid impermeable backsheet and an absorbent system 17 between the topsheet 19 and the backsheet.

As used herein the term "absorbent system" 17 refers to the part of the absorbent core which is effectively absorbing and where absorbent material is present, thereby excluding peripheral portions of the absorbent core where only one or more core wrap layers may be present and no absorbent material is present. The absorbent system may however comprise one or more channels substantially free of absorbent material, which are formed inboard of a perimeter of said absorbent system. The absorbent system 17 has a periphery defined by longitudinal side edges 171,171' and lateral side edges 172,172', which encircles the outermost positioning of absorbent material.

The absorbent article 10 includes barrier cuffs 18,18' extending in a longitudinal direction from the front waist region 12 to the back waist region 14 along the topsheet 19. Each barrier cuff 18,18' has a front end 184 at the front waist region 12 and a back end 185 at the back waist region 14; a proximal edge 181 and a distal edge 182 connecting the front end 184 and the back end 185; an inboard surface and an outboard surface 183; and cuff elastic(s) 186, for spacing the distal edge 182 away from the topsheet 19 top surface.

The diaper 10 additionally includes one or more attachment means for securing closed the front and back ends 184 and 185 of each barrier cuff 18,18'. Attachment means may include adhesive bonds such as an adhesive glue bead, thermal bonds, mechanical bonds, pressure bonds, ultrasonic bonds, combinations of these, or the like. The areas in which the attachment means are disposed are designated the front tack-down regions 187 and the back tack-down regions 188; they are diagonally hatched in FIG. 1. Therefore each barrier cuff distal edge 182 is attached to the topsheet 19 at a tack-down region 187,188 in the front waist region 12 and in the back waist region 14. A tack-down region 187,188 begins at an outer tack-down edge 189 located in proximity of the chassis lateral outer edge 16,16' and ends at an inner tack-down edge 190 that is spaced longitudinally from the outer tack-down edge 189.

We have found that a containment pocket could be created in a waist region 12,14 by ensuring that the tack-down region 187,188 does not overlay the absorbent system 17 in said waist region, such that a ratio R between the length A of the tack-down region 187,188 measured parallel to the longitudinal axis Y in said waist region and the distance B between the lateral outer edge 16,16' of the chassis 11 and the lateral side edge 172,172' of the absorbent system 17 measured parallel to the longitudinal axis Y in said waist region is in the range 0.05 to 0.95, all measurements being made whilst the absorbent article 10 is in a stretched, laid-flat configuration. Distance B in the front waist region 12 is herein measured outside of any navel cut which could be present in the lateral outer edge 16' of the chassis 11.

Contrary to some previous attempts to avoid waist leakage by lengthening the absorbent system into the waist region to increase absorbency in said region, which inevitably caused the tack-down region to come over the absorbent system even more, we have unexpectingly found that increasing the distance between the tack-down region and the absorbent system was rather the solution.

Advantageously, the ratio R is in the range 0.60 to 0.90, preferably in the range 0.65 to 0.85, more preferably in the range 0.70 to 0.80. These ranges may advantageously allow the pocket to be large enough to contain exudates, for example liquid stool in the back waist region, whilst avoiding a too long chassis or a too short absorbent system.

The present containment pocket is particularly advantageous at the back of the diaper, in particular to contain semi-solid fecal material, such as low viscosity fecal material, which can be prevalent with younger children. In preferred embodiments, like the one illustrated in FIG. 1, only the back waist region shows a ratio R within the herein defined ranges.

In advantageous embodiments, the back waist region includes an elastic waistband 20 (vertically hatched in FIG. 1) extending along an axis parallel to the transverse axis. An advantage of this arrangement may be improved comfort and adherence onto the wearer's torso that further aids in reducing the risk of leakage. Furthermore, we have found that the combination of a ratio R within the herein defined ranges in the back waist region and an elastic waistband in the back waist region, provides a containment pocket in the back waist region which may be even more efficient to retain exudates, showing for example better results than the same article without the elastic waistband when the pocket is small and high volumes of body exudates are involved. In addition, the combination of the herein defined ratio R with an elastic waistband in a same waist region provides an enhanced visual appearance of the containment pocket, giving to the buyer or caregiver even more trust in the efficiency of the absorbent article and providing an appealing distinction over other absorbent articles which do not offer such containment pocket.

The elastic waistband may be applied onto the topsheet or may be applied to other components of the absorbent article, such as backsheet, or may be sandwiched between different layers of the absorbent article, such as between the topsheet and backsheet. The elastic waistband may be an elastomeric, cloth-like, nonwoven fibrous material, such as an elastomeric stretch bonded laminate web or an elastomeric meltblown web. In some embodiments, the elastic waistband is constructed by stretching an elastic material and, while the elastic material is in a stretched state, bonding the elastic material to at least one precursor web in such a manner that, when tension is released on the resulting elastic waistband, the entire elastic waistband contracts. Gathers or pleats may be formed in the elastic waistband. Precursor web may comprise nonwovens, films, or composites thereof, and may have a basis weight in the range of 10 gsm to 30 gsm. The elastic material may comprise an elastomeric film or one or more elastomeric strands or ribbons.

In an embodiment (not shown), the absorbent article may additionally comprise at least one transversal barrier cuff positioned in at least one of the front and back waist regions and extending parallel to the transverse axis. The barrier cuff may supplement the containment pocket of the present disclosure for collection of exudates therein. It may comprise one or more elastics positioned at an open end thereof being distal from a closed end thereof. Advantageously, when combined with an elastic waistband, the transversal barrier cuff is positioned below the elastic waistband on the skin facing surface of the absorbent article. In this arrangement, the elastic waistband provides for a tight closure at a closed end of the pocket aiding the effectiveness of the leakage prevention barrier.

The absorbent article of FIG. 1 comprises a pair of opposed elasticized side panels 40 joined to the chassis 11 at the back waist region 14, each side panel extending out from respective longitudinal outer edges 15,15' of the chassis at the back waist region 14, and one or more fastening members 50 joined to each of said opposed elasticized side panels 40 releasably joinable to a garment facing side of the front waist region 12, wherein opposing fastening members 50 are positioned on a fastener axis F, which is a centreline axis dividing the fastening members 50 into two equal halves and extending parallel to the transverse axis X.

In preferred embodiments, a ratio Q between the length A of the tack-down region 188 and a distance C between the lateral outer edge 16,16' of the chassis 11 and the fastener axis F is in the range 0.8 to 1.4, preferably in the range 1 to 1.3, all measurements being again made whilst the absorbent article is in a stretched, laid-flat configuration. This may help further in ensuring snugly fit around the waist of the wearer.

When the diaper is as worn by a user (i.e. when the cuff elastics 186 are contracted and the barrier cuffs 18,18' raised), a containment pocket forms itself in a region of the chassis herein called pocket zone.

The extent of the back pocket zone 30, present in the back waist region, may be defined as a region of the chassis bounded by the fastener axis F, by the proximal edges 181 of the barrier cuffs 18,18' and by the lateral side edge 172 of the absorbent system 17. The longitudinal extension of the back pocket zone 30, corresponding to the difference between distance B and distance C, is within the range 15 to 60 mm, preferably in the range 20 to 50 mm.

The formation of the containment pocket is rendered possible because the chassis in the pocket zone may be very flexible since it does not include too many layers of material, in particular no absorbent material and preferably no acquisition-distribution layer and/or no curly fibres (i.e. chemically modified cross-linked cellulose fibres) and because of the tension in the barrier cuffs. Therefore the chassis advantageously consists essentially of the topsheet and the backsheet in at least 50%, preferably 60%, more preferably 70%, still more preferably 80%, most preferably 90% of the surface area of the back pocket zone 30. A portion of the elastic waistband 20, when present, may be included in the back pocket zone 30, but preferably not exceeding 50% of the surface area of the back pocket zone 30. This may allow the back pocket zone to remain flexible enough to create the containment pocket once worn.

A theoretical volume of the containment pocket may be calculated as a half cylinder, wherein the height of the cylinder is the distance between the proximal edges 181 of the barrier cuffs 18,18', and its arc-shaped periphery length corresponds to B - C. Preferably, the containment pocket has a theoretical volume in the range 2 to 70 cm³, more preferably in the range 5 to 60 cm³, still more preferably in the range 7 to 50 cm³.

Advantageously, the absorbent article may include visual indicia in the region of the pocket zone to enhance its noticeability and/or visibility, preferably from the outside of the absorbent article. This may allow for visual distinction of the article compared to articles which do not have a pocket, and/or may give to the buyer or caregiver more trust in the efficiency of the absorbent article. For example the backsheet may have a different colour or design in the pocket zone. Another example can be when an elastic waistband is present and when the core wrap does not extend beyond the absorbent system, to use a coloured waistband and a coloured core wrap, leaving the pocket zone uncoloured.

### EXAMPLES and COMPARATIVE EXAMPLE

Exemplary diapers according to embodiments of the herein disclosure were manufactured in size Mini (size 2) and tested according to the Bucket Test herein described.

Exemplary diapers include, from top to bottom :
- Barrier cuffs available from PFNonwovens Czech s.r.o. under reference SMS SOFT 17GSM PHILIC WHI
- A nonwoven topsheet available from Sandler AG under reference 01307-004
- A nonwoven acquisition and distribution layer available from Union Industries Spa under reference T3000PIWT
- An absorbent core consisting essentially of
   ∘ A nonwoven top core wrap layer available from Union Polska Sp. Z. o.o. under reference D0801PIW
   ∘ An absorbent system consisting of fluff, and SAP available from Ekotec Industrietechnik GmbH, in a ratio 1:1
   ∘ A nonwoven bottom core wrap layer available from PFNonwovens Czech s.r.o. under reference SMS 8GSM PHILIC WHI
   the absorbent core comprising a channel as described in EP3562455
- A backsheet consisting essentially of a PE film and a nonwoven outer backsheet

In some of the exemplary diapers (Ex. 4 to 6), an elastic waistband available from Fitesa Retsag KFT under reference EXTRAFLEX CEX836RT-39200 is present in the back waist region, as schematically drawn in Fig. 1.

The exemplary diapers all show a back pocket zone where the chassis of the diapers allows a containment pocket to be created when the cuff elastics are contracted and the barrier cuffs raised.

### Comparative example (not in accordance with the present disclosure)

Exemplary diapers were compared to a commercially available diaper manufactured by Procter & Gamble, sold under the trademark Pampers^{®} baby-dry^{™}, in size 2, purchased in July 2022 in Mayen (Germany), and having the following code on the bag: 28674499KK 36 02.03 08/03/22 MADE in Germany E.

The absorbent system of this comparative example diaper generally consists of SAP and fluff, and, in a back region, consists of SAP (without fluff). This comparative example is one example of previous attempts to avoid waist leakage by lengthening the absorbent system into the back waist region to increase absorbency in said region. In the present case, SAP are enclosed between upper and lower core wraps until 15 mm from the back lateral outer edge of the chassis.

**Table 1 summarises the different dimensions in mm measured on the diapers of the examples (Ex. 1 to Ex. 6) and of the comparative example (Comp. Ex. 1). These dimensions are an average taken on 5 different diapers having the same configuration.**

| Table 1 | Ex. 1 | Ex. 2 | Ex. 3 | Ex. 4 | Ex. 5 | Ex. 6 | Comp. Ex. 1 |
|---|---|---|---|---|---|---|---|
| EWB | No | No | No | Yes | Yes | Yes | No |
| A | 54 | 66 | 61 | 58 | 68 | 69 | 75 |
| B | 77 | 86 | 92 | 77 | 90 | 93 | 15 |
| R = A/B | 0.70 | 0.77 | 0.66 | 0.75 | 0.76 | 0.74 | 5 |
| C | 52 | 54 | 53 | 51 | 62 | 53 | 48 |
| B-C | 25 | 32 | 39 | 26 | 28 | 40 | No pocket zone |
| Q = A/C | 1.04 | 1.22 | 1.15 | 1.14 | 1.10 | 1.30 | 1.54 |

**Table 2 shows the results of the Bucket Test with various quantities of low viscosity fecal material substitute.**

| Table 2 | Ex. 1 | Ex. 2 | Ex. 3 | Ex. 4 | Ex. 5 | Ex. 6 | Comp. Ex. 1 |
|---|---|---|---|---|---|---|---|
| 10 ml | V | V | V | V | V | V | X |
| 30 ml | V | - | - | V | - | - | O |
| 50 ml | X | V | V | V | V | V | O |

| | | | | | | | |
|---|---|---|---|---|---|---|---|
| "V" indicates that the low viscosity fecal material substitute stays trapped within the containment pocket. "X" indicates that the low viscosity material substitute passes over the containment pocket (when present) and nearly reaches the back lateral outer edge of the chassis, but without exiting from the diaper. "O" indicates that the low viscosity material substitute gets out of the diaper, passing over the back lateral outer edge of the chassis. "-" indicates that the test was not performed. | | | | | | | |

It can be seen from these results that a containment pocket according to the present disclosure, positioned in the back waist region, allows to reliably retain low viscosity fecal material and avoid leakage. According to the expected quantity of fecal material, leakage can be avoided by ensuring a larger pocket zone and/or adding an elastic waistband. Example 1 with small pocket zone (B-C of about 25 mm) and large quantity of low viscosity fecal material substitute (50 ml) shows some fecal material passing over the containment pocket and nearly getting out of the diaper, but no leakage out of the diaper. Examples 2 and 3 show that this can be improved by increasing the pocket zone size (respectively to about 30 mm and 40 mm). Example 4 shows that this can be improved by adding an elastic waistband, while keeping the pocket zone size identical (B-C of about 26 mm).

Comparative example 1 shows that lengthening the absorbent system is not a solution to fully avoid leakage. Only with 10 ml of low viscosity fecal material substitute, does the comparative diaper not show leakage out of the diaper, but still material nearly getting out of the diaper. In other cases leakage occur. Moreover lengthening the absorbent system increases the cost of the absorbing article, for example the cost of extra SAP to be incorporated in the back region of this comparative example is not negligible.

These examples and comparative example confirm that ensuring a containment pocket according to the present disclosure retains or improves high performance against leakage of exudates, whilst being of simpler and/or less costly construction.

### BUCKET TEST

The following is a description of the Bucket Test used in the examples and comparative example described herein.

A diaper is positioned, open and topsheet facing upwards, within a bucket having an opening of around 20 cm. It is maintained in this position with 4 metal binder clips positioned on the edge of the bucket and the lateral edges of the diaper, in the tack-down regions. The diaper is let hanging within the bucket, without touching the bottom of the bucket.

The bucket is left freely hanging, with the possibility to lift it at 90°, i.e. to the horizontal. A plate is left below the bucket to collect low viscosity fecal material substitute potentially getting out of the diaper during the test.

Test with 10 ml of low viscosity fecal material substitute:
The bucket is maintained at a 90° position, with the back waist region of the diaper downwards. 10 ml of low viscosity fecal material substitute is applied with a graduated syringe at a position along the longitudinal centreline of the diaper, at a distance from the chassis lateral back edge which corresponds to 0.31 of the length of the diaper. The low viscosity fecal material substitute moves with gravity. The 90° position is maintained until the substitute does not move significantly any more.

Test with 30 ml or 50 ml of low viscosity fecal material substitute:
30 or 50 ml of low viscosity fecal material substitute is measured in a beaker and poured at a position where the longitudinal and lateral centrelines of the diaper cross each other. Immediately after, the bucket is flipped and maintained to a 90° position, with the back waist region of the diaper downwards. The low viscosity fecal material substitute moves with gravity. The 90° position is maintained until the substitute does not move significantly any more.

### PREPARATION OF LOW VISCOSITY FECAL MATERIAL SUBSTITUTE

The following is a description of the fecal material substitute used in the Bucket Test described herein.

Ingredients:
Stock mixture:
   - Activia^{®} Natur yoghurt
   - Egg white (from egg with size M)
   - Turmeric powder
   - 1.3 % w/w dish soap solution, e.g. 1.28 g Pril^{®} Kraftgel Ultra Plus + 97.20 g deionised water
Swelling agent:
   - Cellulose powder, e.g. 2-Hydroxyethyl cellulose from Aldrich^{®} chemistry

Preparation of the stock mixture:
Mix yoghurt, egg white, turmeric powder and 1.3 % dish soap solution with the following weight ratios:
   □ Egg white / yoghurt 1:1.4370
   □ Egg white / turmeric powder 1:0.0756
   □ Egg white / dish soap solution 1:0.0252

Mix the ingredients until the preparation is homogeneously yellow: first mix vigorously by hand using a spoon, and once the mixture looks homogeneous, use a magnetic stirrer.

Preparation of the final mixture:
Divide the stock mixture: about 1/5 in a small container and 4/5 in a larger container. The 1/5 portion will be used to adjust the viscosity later.

In the larger container, add cellulose powder in a weight proportion of 1,55 % of the stock mixture and mix with a magnetic stirrer. The thickening process may take several hours, at least 4 hours. If possible stir a few times during the thickening process.

The final mixture is ready when the viscosity is at η = 400 ± 20 cP. If the mixture is being used for several hours, it is advised to measure the viscosity on a regular basis to double-check it, for example every 2 hours.

In case the viscosity goes over 400 cP, carefully add some of the stock mixture from the small container to dilute the mixture and reduce viscosity.

Viscosity may be measured with a viscotester, in particular a HAAKE Viscotester C (L-Version) from ThermoFisher Scientific, with Spindel L2, set on the viscosity program. Since the mixture is drying fast, also on the spindle, clean up device each time before re-testing the viscosity of the mixture.

## Claims

1. An absorbent article (10) comprising:
a chassis (11) including a topsheet (19), a backsheet joined to the topsheet (19), and an absorbent system (17) located between the topsheet (19) and the backsheet, the chassis (11) having a front waist region (12), a back waist region (14), a crotch region (13) located between the front waist region (12) and the back waist region (14), a longitudinal axis (Y) extending through the front and back waist regions (12,14) and a transverse axis (X) substantially perpendicular to the longitudinal axis (Y), the chassis (11 having a periphery defined by longitudinal outer edges (15,15') and lateral outer edges (16,16)', the absorbent system (17) having a periphery defined by longitudinal side edges (171,171') and lateral side edges (172,172'),
barrier cuffs (18,18') extending in a longitudinal direction from the front waist region (12) to the back waist region (14) along the topsheet (19), each barrier cuff (18,18') including a front end (184) at the front waist region (12), a back end (185) at the back waist region (14) and proximal (181) and distal (182) edges connecting the front end (184) and the back end (185), each barrier cuff distal edge (182) being attached to the topsheet (19) at a tack-down region (187,188) in the front waist region (12) and in the back waist region (14);
**characterized in that**, in at least one of the front and back waist regions (12,14), a ratio R between a length A of the tack-down region (187,188) measured parallel to the longitudinal axis (Y) in said waist region and a distance B between the lateral outer edge (16,16') of the chassis (11) and the lateral side edge (172,172') of the absorbent system (17) measured parallel to the longitudinal axis (Y) in said waist region is in the range 0.05 to 0.95, all measurements being made whilst the absorbent article is in a stretched, laid-flat configuration.

2. An absorbent article according to claim 1, wherein ratio R is in the range 0.05 to 0.95 in the back waist region (14).

3. An absorbent article according to claim 1 or claim 2, wherein ratio R is in the range 0.60 to 0.90, preferably in the range 0.65 to 0.85, more preferably in the range 0.70 to 0.80.

4. An absorbent article according to any of the preceding claims, wherein the back waist region (14) includes an elastic waistband (20) extending along an axis parallel to the transverse axis (X).

5. An absorbent article according to claim 4, wherein the elastic waistband (20) comprises an elastomeric film or one or more elastomeric strands or ribbons.

6. An absorbent article according to any of the preceding claims, wherein at least one of the front and back waist regions (12,14) comprise at least one transversal barrier cuff.

7. An absorbent article according to any of the preceding claims, wherein the absorbent article comprises a pair of opposed elasticized side panels (40) joined to the chassis (11) at the back waist region (14) and one or more fastening members (50) joined to each of said opposed elasticized side panels (40), wherein opposing fastening members (50) are positioned on a fastener axis (F) which is a centreline axis dividing the fastening members (50) into two equal halves and extending parallel to the transverse axis (X), **characterised in that** a ratio Q between the length A of the tack-down region (188) and a distance C between the lateral outer edge (16,16') of the chassis (11) and the fastener axis (F) is in the range 0.8 to 1.4, preferably in the range 1 to 1.3.

8. An absorbent article according to claim 7, wherein the chassis (11) comprises a back pocket zone (30) bounded by the fastener axis (F), by the proximal edges (181) of the barrier cuffs (18,18') and by the lateral side edge (172) of the absorbent system (17).

9. An absorbent article according to claim 8, wherein a longitudinal extension of the back pocket zone (30), corresponding to the difference between distance B and distance C, is within the range 15 to 60 mm, preferably in the range 20 to 50 mm.

10. An absorbent article according to claim 8 or claim 9, wherein the absorbent article includes visual indicia in the region of the back pocket zone (30) to enhance its noticeability.

11. An absorbent article according to any of claims 8 to 10, wherein the chassis (11) consists essentially of the topsheet (19) and the backsheet in at least 50% of the surface area of the back pocket zone (30).
